# EUROPEAN PATENT APPLICATION

(11) **EP 1 561 816 A1**
(43) Date of publication of application: **10.08.2005**
(21) Application number: 03758984.3
(22) Date of filing: 28.10.2003
(51) Int. Cl.: C12N 15/12, C07K 16/18, C12Q 1/68

(54) **METHODS FOR DETERMINING GENETIC RESISTANCE OF PIGS TO DISEASES CAUSED BY RNA VIRUSES**

(30) Priority: 28.10.2002 JP 2002313076
(71) Applicant: National Institute of Agrobiological Sciences, Tsukuba-shi, Ibaraki 305-8602 (JP)
(72) Inventor: MITSUHASHI, Tadayoshi, Tsukuba-shi, Ibaraki 305-0035 (JP)
(74) Representative: Bassil, Nicholas Charles
(86) International application number: PCT/JP2003/013767
(87) International publication number: WO 2004/038022

(57) **Abstract**

The inventors investigated the impact of an 11-bp deletion in the swine Mx1 gene on the ability to suppress propagation of influenza viruses belonging to the myxovirus family, and revealed that the deletion led to a complete loss of the ability to suppress viral propagation. Through the detection of the 11-bp deletion, pigs can be examined for their resistance to RNA viruses such as influenza viruses and the virus that causes PRRS.

SEQUENCE LISTING

<110> National Institute of Agrobiological Sciences

<120> METHODS FOR DETERMINING GENETIC RESISTANCE OF PIGS TO DISEASES CAUSED
      BY RNA VIRUSES

<130> MOA-A0214P

<140>
<141>

<150> JP 2002-313076
<151> 2002-10-28

<160> 10

<170> PatentIn Ver. 2.1

<210> 1
<211> 2545
<212> DNA
<213> Sus scrofa

<220>
<221> CDS
<222> (101)..(2092)

<220>
<221> polyA_signal
<222> (2517)..(2522)

<400> 1
gtaagtgtgg gagaacagcc ctgcatttct gctgacgggt caacgtcaca gcgtcaaaga 60

aaaggaaggt acatttcagc tgaactgatc aaggaggaag atg gtt tat tcc agc    115
                                            Met Val Tyr Ser Ser
                                              1               5

tgt gaa agt aaa gaa cct gat tca gtt tct gca tcc aat cac ctg tta    163
Cys Glu Ser Lys Glu Pro Asp Ser Val Ser Ala Ser Asn His Leu Leu
                 10                  15                  20

cta aat ggg aat gat gaa ttg gtg gag aaa agt cac aaa aca ggg cct    211
Leu Asn Gly Asn Asp Glu Leu Val Glu Lys Ser His Lys Thr Gly Pro
             25                  30                  35

gag aac aac ctg tac agc cag tac gag gag aaa gtg cgg ccc tgc atc    259
Glu Asn Asn Leu Tyr Ser Gln Tyr Glu Glu Lys Val Arg Pro Cys Ile
         40                  45                  50

gac ctc atc gac tca ctg cgg gcc ctg ggc gtg gag cag gac ctg gcc    307
Asp Leu Ile Asp Ser Leu Arg Ala Leu Gly Val Glu Gln Asp Leu Ala
     55                  60                  65

ctg ccc gcc atc gcc gtc atc ggg gac cag agt tcg ggc aag agc tcc    355
Leu Pro Ala Ile Ala Val Ile Gly Asp Gln Ser Ser Gly Lys Ser Ser
 70                  75                  80                  85

gtg ctg gag gcc ctg tcg ggg gtc gct ctc ccc aga ggc agc gga att    403
Val Leu Glu Ala Leu Ser Gly Val Ala Leu Pro Arg Gly Ser Gly Ile
                 90                  95                 100

gtg aca aga tgc cct ctt gtg ctg aaa ttg aaa aaa ctc gtg aac gaa    451
Val Thr Arg Cys Pro Leu Val Leu Lys Leu Lys Lys Leu Val Asn Glu
            105                 110                 115

gaa gac gaa tgg aag ggc aaa gtc agt tac cgg gac agc gag att gag    499
Glu Asp Glu Trp Lys Gly Lys Val Ser Tyr Arg Asp Ser Glu Ile Glu

        120                 125                 130

ctt tca gat gct tcg cag gtg gaa aag gaa gtc agc gca gcc cag att 547
Leu Ser Asp Ala Ser Gln Val Glu Lys Glu Val Ser Ala Ala Gln Ile
    135                 140                 145

gcc atc gct ggg gaa ggc gtg gga atc agt cat gag cta atc agt ctg 595
Ala Ile Ala Gly Glu Gly Val Gly Ile Ser His Glu Leu Ile Ser Leu
150                 155                 160                 165

gag gtc agc tcc cct cat gtc cca gat ctg acc ctc ata gac ctt cct 643
Glu Val Ser Ser Pro His Val Pro Asp Leu Thr Leu Ile Asp Leu Pro
                170                 175                 180

ggc atc acc agg gta gct gta ggc aat cag cca tac gac atc gaa tac 691
Gly Ile Thr Arg Val Ala Val Gly Asn Gln Pro Tyr Asp Ile Glu Tyr
            185                 190                 195

cag atc aag tct ctg atc aag aag tac atc tgt aag cag gag acc atc 739
Gln Ile Lys Ser Leu Ile Lys Lys Tyr Ile Cys Lys Gln Glu Thr Ile
        200                 205                 210

aac ttg gtg gtg gtc ccc tgt aac gtg gac att gcc acc acg gag gcg 787
Asn Leu Val Val Val Pro Cys Asn Val Asp Ile Ala Thr Thr Glu Ala
    215                 220                 225

ctg cgc atg gcc cag gag gtg gac ccc gaa gga gac agg acc atc ggg 835
Leu Arg Met Ala Gln Glu Val Asp Pro Glu Gly Asp Arg Thr Ile Gly
230                 235                 240                 245

atc ttg acg aag ccg gat ctg gtg gac aaa ggc act gag gac aag ata 883
Ile Leu Thr Lys Pro Asp Leu Val Asp Lys Gly Thr Glu Asp Lys Ile
                250                 255                 260

gtg gac gtg gcg aga aac ctg gtc ttc cac ctg aag aag ggc tac atg 931
Val Asp Val Ala Arg Asn Leu Val Phe His Leu Lys Lys Gly Tyr Met
            265                 270                 275

att gtc aag tgc agg ggc cag cag gac atc cag gag cag ctg agc ctg 979
Ile Val Lys Cys Arg Gly Gln Gln Asp Ile Gln Glu Gln Leu Ser Leu
        280                 285                 290

gcc aag gcc ctg cag aag gag cag gcc ttc ttt gaa aac cac gca cat 1027
Ala Lys Ala Leu Gln Lys Glu Gln Ala Phe Phe Glu Asn His Ala His
    295                 300                 305

ttc agg gat ctt ctg gag gaa ggg cgg gcc acg atc ccc tgc ctg gca 1075
Phe Arg Asp Leu Leu Glu Glu Gly Arg Ala Thr Ile Pro Cys Leu Ala
310                 315                 320                 325

gaa aga ctg acc tct gaa ctc atc atg cac atc tgt aaa act ctg ccc 1123
Glu Arg Leu Thr Ser Glu Leu Ile Met His Ile Cys Lys Thr Leu Pro
                330                 335                 340

ctg tta gaa aac caa ata aaa gag agt cac cag aaa ata aca gag gag 1171
Leu Leu Glu Asn Gln Ile Lys Glu Ser His Gln Lys Ile Thr Glu Glu
            345                 350                 355

tta cag aag tat ggc tcc gat att cca gag gat gaa agc ggg aag atg 1219
Leu Gln Lys Tyr Gly Ser Asp Ile Pro Glu Asp Glu Ser Gly Lys Met
        360                 365                 370

ttt ttt ctg ata gat aaa atc gat gca ttt aat agt gat atc act gct 1267
Phe Phe Leu Ile Asp Lys Ile Asp Ala Phe Asn Ser Asp Ile Thr Ala
    375                 380                 385

ttg ata caa gga gag gaa ctg gtg gtg gag tac gag tgt cgg ctg ttt 1315
Leu Ile Gln Gly Glu Glu Leu Val Val Glu Tyr Glu Cys Arg Leu Phe
390                 395                 400                 405

acc aag atg cga aat gag ttc tgc aga tgg agt gct gtg gtt gaa aag 1363
Thr Lys Met Arg Asn Glu Phe Cys Arg Trp Ser Ala Val Val Glu Lys
                410                 415                 420

aat ttc aaa aat ggt tat gac gcc ata tgt aaa caa atc cag ctc ttc 1411
Asn Phe Lys Asn Gly Tyr Asp Ala Ile Cys Lys Gln Ile Gln Leu Phe
            425                 430                 435

gaa aat cag tac agg ggg aga gag ttg cca ggg ttt gtg aat tat aag 1459
Glu Asn Gln Tyr Arg Gly Arg Glu Leu Pro Gly Phe Val Asn Tyr Lys
        440                 445                 450

aca ttt gaa acc atc att aag aag cag gtc agt gtc ctg gaa gag cca 1507
Thr Phe Glu Thr Ile Ile Lys Lys Gln Val Ser Val Leu Glu Glu Pro
    455                 460                 465

gcc gtg gac atg ctg cac aca gtg act gat tta gtc cgg ctc gcc ttc 1555
Ala Val Asp Met Leu His Thr Val Thr Asp Leu Val Arg Leu Ala Phe
470                 475                 480                 485

aca gat gtt tca gaa aca aat ttt aat gaa ttt ttc aac ctc cac aga 1603
Thr Asp Val Ser Glu Thr Asn Phe Asn Glu Phe Phe Asn Leu His Arg
                490                 495                 500

act gcc aag tcc aaa att gaa gac att aaa tta gaa caa gaa aaa gaa 1651
Thr Ala Lys Ser Lys Ile Glu Asp Ile Lys Leu Glu Gln Glu Lys Glu
            505                 510                 515

gct gag acg tcg atc cgg ctc cac ttc caa atg gag cag atc gtg tac 1699
Ala Glu Thr Ser Ile Arg Leu His Phe Gln Met Glu Gln Ile Val Tyr
        520                 525                 530

tgc cag gac cag gtc tat cgg ggc gcg ctg cag aag gtc aga gag aag 1747
Cys Gln Asp Gln Val Tyr Arg Gly Ala Leu Gln Lys Val Arg Glu Lys
    535                 540                 545

gag gcg gaa gaa gaa aag aac aga aaa tca aac cag tac ttt ctg tcg 1795
Glu Ala Glu Glu Glu Lys Asn Arg Lys Ser Asn Gln Tyr Phe Leu Ser
550                 555                 560                 565

tcg ccg gcc ccc tcc tca gac ccc tcc ata gcc gag atc ttt cag cac 1843
Ser Pro Ala Pro Ser Ser Asp Pro Ser Ile Ala Glu Ile Phe Gln His
                570                 575                 580

ctg att gcc tac cat cag gag gtc ggc aag cgc atc tcc agc cac atc 1891
Leu Ile Ala Tyr His Gln Glu Val Gly Lys Arg Ile Ser Ser His Ile
            585                 590                 595

cct ctg atc atc cag ttc ttc atc ctc cgg acc ttt ggg cag cag ctg 1939
Pro Leu Ile Ile Gln Phe Phe Ile Leu Arg Thr Phe Gly Gln Gln Leu
        600                 605                 610

cag aag agc atg ctg cag ctg ctg cag aac aag gac caa tac gac tgg 1987
Gln Lys Ser Met Leu Gln Leu Leu Gln Asn Lys Asp Gln Tyr Asp Trp
    615                 620                 625

ctc ctg agg gag cgc agt gac acc agc gac aag agg aag ttc ctg aag 2035
Leu Leu Arg Glu Arg Ser Asp Thr Ser Asp Lys Arg Lys Phe Leu Lys
630                 635                 640                 645

gag cgg ctg atg cgg ctg acc cag gct cgg cgc cgg ctc gcc aag ttc 2083
Glu Arg Leu Met Arg Leu Thr Gln Ala Arg Arg Arg Leu Ala Lys Phe
                650                 655                 660

cca ggc tga accggactct ccaggcggcc cggggtctcc agggcacgtc 2132
Pro Gly

tccaggcaac gaggaccaac ctccttccct aacagactag catcatgagc tcctgtttcg 2192

cacatcctcc tgtggttagt agactctaaa gccaccgtcc ctgctgttag tggctgagga 2252

cttagcaaga agctgtgata agcacgctgg ctgcaagcat caggccattt acttgaatga 2312

gccccgccaa cgcttcgcct cccgcgcctc tctccatccc tctctccatc cttctctcca 2372

tccctgtata ggatactggt ccccgcatag catcatagaa gggtcattct ggtttctgta 2432

caagcctttc acgcccaatg tcttaggggc attacagcca cctgtgtgga tggatgcaca 2492

tagaagccta tttcttttat ttgtaataaa cttggttcta ccagcaaaaa aaa 2545

<210> 2
<211> 663
<212> PRT
<213> Sus scrofa

<400> 2
Met Val Tyr Ser Ser Cys Glu Ser Lys Glu Pro Asp Ser Val Ser Ala
1               5                   10                  15

Ser Asn His Leu Leu Leu Asn Gly Asn Asp Glu Leu Val Glu Lys Ser
            20                  25                  30

His Lys Thr Gly Pro Glu Asn Asn Leu Tyr Ser Gln Tyr Glu Glu Lys
        35                  40                  45

Val Arg Pro Cys Ile Asp Leu Ile Asp Ser Leu Arg Ala Leu Gly Val
    50                  55                  60

Glu Gln Asp Leu Ala Leu Pro Ala Ile Ala Val Ile Gly Asp Gln Ser
65                  70                  75                  80

Ser Gly Lys Ser Ser Val Leu Glu Ala Leu Ser Gly Val Ala Leu Pro
                85                  90                  95

Arg Gly Ser Gly Ile Val Thr Arg Cys Pro Leu Val Leu Lys Leu Lys
            100                 105                 110

Lys Leu Val Asn Glu Glu Asp Glu Trp Lys Gly Lys Val Ser Tyr Arg
        115                 120                 125

Asp Ser Glu Ile Glu Leu Ser Asp Ala Ser Gln Val Glu Lys Glu Val
    130                 135                 140

Ser Ala Ala Gln Ile Ala Ile Ala Gly Glu Gly Val Gly Ile Ser His
145                 150                 155                 160

Glu Leu Ile Ser Leu Glu Val Ser Ser Pro His Val Pro Asp Leu Thr
                165                 170                 175

Leu Ile Asp Leu Pro Gly Ile Thr Arg Val Ala Val Gly Asn Gln Pro
            180                 185                 190

Tyr Asp Ile Glu Tyr Gln Ile Lys Ser Leu Ile Lys Lys Tyr Ile Cys
        195                 200                 205

Lys Gln Glu Thr Ile Asn Leu Val Val Val Pro Cys Asn Val Asp Ile
    210                 215                 220

Ala Thr Thr Glu Ala Leu Arg Met Ala Gln Glu Val Asp Pro Glu Gly
225                 230                 235                 240

Asp Arg Thr Ile Gly Ile Leu Thr Lys Pro Asp Leu Val Asp Lys Gly
                245                 250                 255

Thr Glu Asp Lys Ile Val Asp Val Ala Arg Asn Leu Val Phe His Leu

            260                 265                 270
Lys Lys Gly Tyr Met Ile Val Lys Cys Arg Gly Gln Gln Asp Ile Gln
        275                 280                 285
Glu Gln Leu Ser Leu Ala Lys Ala Leu Gln Lys Glu Gln Ala Phe Phe
    290                 295                 300
Glu Asn His Ala His Phe Arg Asp Leu Leu Glu Glu Gly Arg Ala Thr
305                 310                 315                 320
Ile Pro Cys Leu Ala Glu Arg Leu Thr Ser Glu Leu Ile Met His Ile
                325                 330                 335
Cys Lys Thr Leu Pro Leu Leu Glu Asn Gln Ile Lys Glu Ser His Gln
            340                 345                 350
Lys Ile Thr Glu Glu Leu Gln Lys Tyr Gly Ser Asp Ile Pro Glu Asp
        355                 360                 365
Glu Ser Gly Lys Met Phe Phe Leu Ile Asp Lys Ile Asp Ala Phe Asn
    370                 375                 380
Ser Asp Ile Thr Ala Leu Ile Gln Gly Glu Glu Leu Val Val Glu Tyr
385                 390                 395                 400
Glu Cys Arg Leu Phe Thr Lys Met Arg Asn Glu Phe Cys Arg Trp Ser
                405                 410                 415
Ala Val Val Glu Lys Asn Phe Lys Asn Gly Tyr Asp Ala Ile Cys Lys
            420                 425                 430
Gln Ile Gln Leu Phe Glu Asn Gln Tyr Arg Gly Arg Glu Leu Pro Gly
        435                 440                 445
Phe Val Asn Tyr Lys Thr Phe Glu Thr Ile Ile Lys Lys Gln Val Ser
    450                 455                 460
Val Leu Glu Glu Pro Ala Val Asp Met Leu His Thr Val Thr Asp Leu
465                 470                 475                 480
Val Arg Leu Ala Phe Thr Asp Val Ser Glu Thr Asn Phe Asn Glu Phe
                485                 490                 495
Phe Asn Leu His Arg Thr Ala Lys Ser Lys Ile Glu Asp Ile Lys Leu
            500                 505                 510
Glu Gln Glu Lys Glu Ala Glu Thr Ser Ile Arg Leu His Phe Gln Met
        515                 520                 525
Glu Gln Ile Val Tyr Cys Gln Asp Gln Val Tyr Arg Gly Ala Leu Gln
    530                 535                 540
Lys Val Arg Glu Lys Glu Ala Glu Glu Glu Lys Asn Arg Lys Ser Asn
545                 550                 555                 560
Gln Tyr Phe Leu Ser Ser Pro Ala Pro Ser Ser Asp Pro Ser Ile Ala
                565                 570                 575
Glu Ile Phe Gln His Leu Ile Ala Tyr His Gln Glu Val Gly Lys Arg
            580                 585                 590
Ile Ser Ser His Ile Pro Leu Ile Ile Gln Phe Phe Ile Leu Arg Thr
        595                 600                 605
Phe Gly Gln Gln Leu Gln Lys Ser Met Leu Gln Leu Leu Gln Asn Lys
    610                 615                 620
Asp Gln Tyr Asp Trp Leu Leu Arg Glu Arg Ser Asp Thr Ser Asp Lys

625                 630                 635                 640

Arg Lys Phe Leu Lys Glu Arg Leu Met Arg Leu Thr Gln Ala Arg Arg
                645                 650                 655

Arg Leu Ala Lys Phe Pro Gly
            660


<210> 3
<211> 23
<212> DNA
<213> Artificial Sequence

<220>
<223> Description of Artificial Sequence:an artificially
      synthesized primer sequence

<400> 3
ctgaaagatc tcggctatgg agg                                          23


<210> 4
<211> 25
<212> DNA
<213> Artificial Sequence

<220>
<223> Description of Artificial Sequence:an artificially
      synthesized primer sequence

<400> 4
aagaagctga gacgtcgatc cggct                                        25


<210> 5
<211> 21
<212> DNA
<213> Artificial Sequence

<220>
<223> Description of Artificial Sequence:an artificially
      synthesized primer sequence

<400> 5
aagcgcatct ccagccacat c                                            21


<210> 6
<211> 20
<212> DNA
<213> Artificial Sequence

<220>
<223> Description of Artificial Sequence:an artificially
      synthesized primer sequence

<400> 6
aagacattgg gcgtgaaagg                                              20


<210> 7
<211> 53
<212> DNA
<213> Artificial Sequence

<220>
<223> Description of Artificial Sequence:an artificially
      synthesized primer sequence

<400> 7
ggggacaagt ttgtacaaaa aagcaggctg tcacagcgtc aaagaaaagg aag        53


<210> 8
<211> 49
<212> DNA
<213> Artificial Sequence

<220>
<223> Description of Artificial Sequence:an artificially
      synthesized primer sequence

<400> 8
ggggaccact ttgtacaaga aagctgggtc cttctatgat gctatgcgg             49


<210> 9
<211> 20
<212> DNA
<213> Artificial Sequence

<220>
<223> Description of Artificial Sequence:an artificially
      synthesized primer sequence

<400> 9
agtgacagga gcgacaagag                                             20


<210> 10
<211> 18
<212> DNA
<213> Artificial Sequence

<220>
<223> Description of Artificial Sequence:an artificially
      synthesized primer sequence

<400> 10
cctggagagt ccggttca                                               18

## Description

### Technical Field

The present invention relates to methods for determining genetic resistance of pigs to diseases caused by RNA viruses.

### Background Art

Mouse studies have shown that the Mx gene encodes a protein that suppresses propagation (inhibits RNA synthesis) of RNA viruses belonging to the orthomyxovirus family (single-stranded linear RNA viruses), such as influenza viruses.

One of the two swine Mx genes is the Mx1 gene, consisting of 14 exons. Some pigs of the Meishan breed have a 3-bp (Ser) deletion in exon 13. On the other hand, some animals of pig breeds that are domesticated in the West and widely used around the world, such as Landrace and Duroc, have an 11-base deletion in the last exon (see Morozumi T. *et al*., "Three types of olymorphisms in exon 14 in porcine Mx1 gene", Biochemical Genetics., (2001) Vol. 39, p. 251-260).

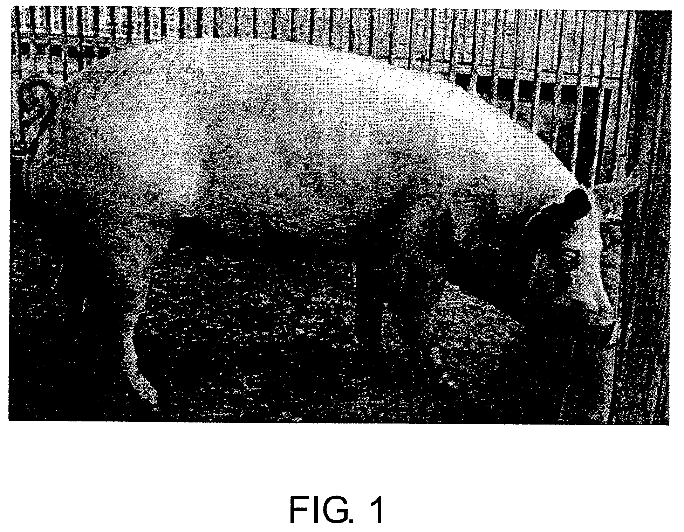

The present inventors have previously reported the presence of Mx1 deletion genotypes and their frequencies (see Morozumi T. *et al*., "Three types of olymorphisms in exon 14 in porcine Mx1 gene", Biochemical Genetics., (2001) Vol. 39, p. 251-260) through collaboration with the STAFF Institute. Among these deletion genotypes, the 11-base deletion was found to exist at a high frequency in Landrace, which is the most commonly used swine breed in the world. Landrace (Fig. 1 photograph) has been widely used to raise three-way crossbreds for fattening/production.

On the other hand, the 11-base deletion has not been found in wild or half-wild species such as the Japanese wild boar and Meishan pig.

The largest worldwide influenza epidemic in history, called the Spanish influenza epidemic, occurred between 1917-1918 and led to 20-40 million deaths. In Japan, more than 300,000 people died. The Spanish influenza epidemic, which started in the Northwest region of the United States, killed approximately 600,000 people in the United States alone. At that time, a cold outbreak in the pig population was also reported. There was also a report on the phylogenetic analysis of an RNA virus obtained from formalin-preserved, paraffin-embedded lung tissue samples taken from soldiers who died during the epidemic. According to the report, the influenza virus belongs to a virus family that is infectious to both humans and pigs. The report also went on to describe that although it is not known whether the change (infection from pig to human or human to pig) was generated in a human host or pig host, it is likely that the virus was an ancestor common to both the human and pig viruses.

Pig farming is still popular in the Northwest region of the United States including the Iowa State. There is no detailed information on pig farming at the time of the outbreak, but if the Landrace breed or its crossbreeds were being raised on a large scale, pig populations having a high percentage of pigs susceptible to influenza viruses may have been acting as a breeding ground for the new virus and resulting in the pandemic. Incidentally, the Duroc Breeder Association already existed in the United States in 1917, and some present-day Durocs are known to have the 11-base deletion.

Farm animals are bred at high densities within limited areas of fixed locations. High-density breeding can mean major damages to production as disease spreads easily through the herd after an initial outbreak. As is evident from examples of influenza transmission from pigs to humans, high-density livestock herds sometimes can act as a breeding ground that transmits diseases to populations across species. To prevent such risks, preventive vaccination is used nowadays, and antibiotics-containing feed is given to animals even if they are in a healthy state. In spite of such preventive measures, great losses in pig production can occur, as exemplified by the outbreak of a new type influenza virus H3N2 in the United States between 1998-2000. Routine administration of antibiotics may result in antibiotics remaining in meat products, and therefore trigger food safety concerns among consumers. However, the preventive measures are necessary for reducing livestock losses from diseases. Genetic information governing potential disease resistance in livestock is required to maintain food safety while retaining or improving the current rate of disease suppression. Nevertheless, such information is still yet to be found.

In addition, so far there is no effective method available for determining genetic resistance of pigs to diseases caused by RNA viruses.

### Disclosure of the Invention

The present invention was achieved under the above circumstances. An objective of the invention is to provide methods for determining genetic resistance of pigs to diseases caused by RNA viruses, in particular, influenza viruses and the causative virus of Porcine Reproductive and Respiratory Syndrome (PRRS).

Some particular livestock and livestock breedsmay have the genetically-inherited disease resistance characteristic of "low productivity, but high resistance to certain diseases". If such a hereditary characteristic of disease resistance can be introduced into breeds with high productivity, breeds of high productivity and high disease resistance can be created. This would enable the reduction of the amounts of antibiotics required. It has been difficult to identify the genetic basis governing such hereditary characteristics at the molecular level. However, the advancement of molecular genetics has accelerated the elucidation of genetic bases of various hereditary characteristics in human and mouse. If the resistance to a particular disease can be related to some of the genetic information in farm animals, disease resistant livestock can be selected and bred based on such genetic information.

In a previous study, the inventors revealed that among domesticated pigs, some have an 11-base deletion in the gene encoding the Mx1 protein, which is responsible for the suppression of myxovirus propagation (already disclosed in a published document). Then, the inventors noticed a lower ratio of animals with homozygous deletion genotype (C) compared to those with heterozygous deletion genotype (C). The homozygous C was conceivably against the survival of pigs based on previous findings that, for example, the percentage of homozygous C present in the Landrace breed was lower than that of the heterozygous C, and in other breeds only heterozygous but no homozygous pigs exist. No homozygous (C/C) pigs were found in the survey of about 40 pigs of white Western breeds, including Landrace, in the old National Institute of Animal Industry in the year 2000.

The inventors understood from pig farming experiences that pigs have poor resistance to respiratory diseases and that this has a huge impact on piglet production.

The present inventors then investigated the effects of the 11-bp deletion in the Mx1 gene on the ability to suppress propagation of influenza viruses (members of the myxovirus family), and revealed that the 11-bp deletants completely lost the ability to suppress virus propagation, and thereby completed the present invention.

More specifically, in Mx1 genes carrying the 11-base deletion in the last exon, the 11-base deletion causes frame shift of codons (single units of triplicate nucleotides), which relocates the stop codon to a much further position downstream and thus dramatically alters the downstream amino acid sequence. The resultant mutant Mx1 protein has a different molecular weight and structure compared to the normal (wild-type) protein, and has probably lost its ability to suppress virus propagation because of that.

Next, the present inventors introduced a normal (wild-type) Mx1 gene (a/a), a mutant Mx1 gene containing a 3-base (3-bp) deletion in exon 13 (b/b), a mutant Mx1 gene containing the 11-base (11-bp) deletion in the last exon (c/c), or an empty vector into murine 3T3 cells which have no Mx1 activity, and performed influenza A virus infection experiments.

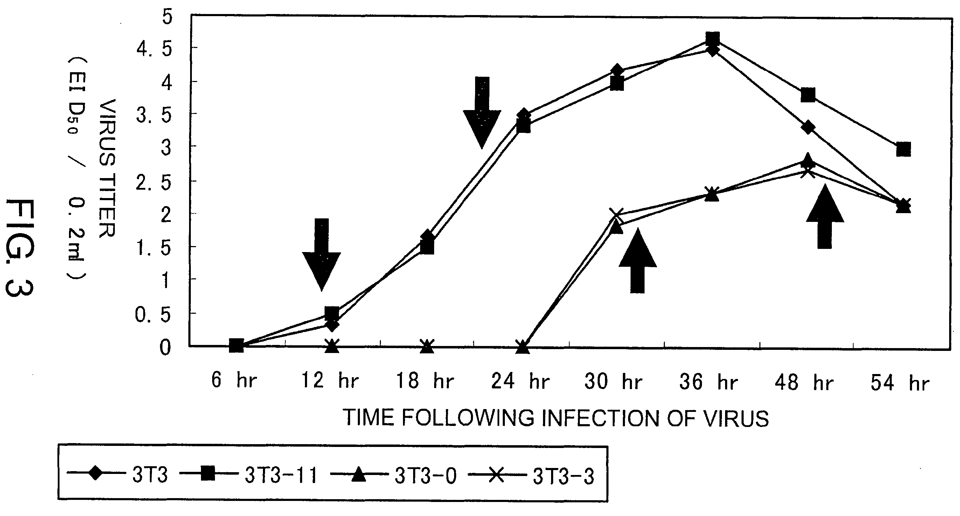

The results showed that the 3-bp deletant had a virus propagation curve comparable to that of the wild type and the deletion did not affect the virus suppression ability. These deletants thus maintained the ability to suppress virus propagation (arrow on the far right of Fig. 3). In contrast, the 11-bp deletant completely lost its ability to suppress virus propagation (arrow on the far left of Fig. 3), and had a virus propagation comparable to that of the empty vector, which was 10-100 times higher than the wild type. Similar results were obtained in a separate experiment of virus infection at a multiplicity of infection that is one order of magnitude smaller (MOI = 1).

In particular, among the domestic pigs used around the world, there are some that carry an Mx1 gene that lacks the ability to suppress viral propagation. According to the experimental results of the present invention, such pigs are expected to have a serious defect in their defense ability during the early phase defense against an RNA virus invasion.

The ability of the swine Mx1 gene to suppress influenza virus propagation is a characteristic acquired during the long history of organisms. Therefore, it is unlikely that the gene is responsible for the suppression of influenza virus propagation only and not for the suppression of other RNA viruses. In the experiments described herein, an influenza virus was used because it is the most problematic virus considered from a practical standpoint of livestock production. Nevertheless, it is unlikely that the gene has suppression ability towards influenza viruses only. It is natural to think that the Mx1 gene also has suppression effects on the propagation of other RNA viruses. The gene is expected to have the ability to suppress propagation of, for example, the corona virus (causative virus of PRRS), and other RNA viruses.

Specifically, the above-described genotype of the swine Mx1 gene can be used to determine the genetic resistance of pigs to diseases caused by RNA viruses (for example, influenza viruses and PRRS causative virus). In addition, it is important to eliminate the 11-base Mx1 deletants not only for the health of livestock, but also for eliminating threats that arise from emergence of new influenza viruses that are infectious to humans.

When an existing breed carries a mutation that leads to partial gene deletion or amino acid substitution, it means that the breed has reproduced while carrying the mutation through many generations, in some cases, for periods as long as tens of thousands of years, from the day when the mutation occurred. In other words, the mutation is considered to have little negative impact on the preservation of life and the continued existence of species. On the other hand, however, lethal genes, which lead to the termination of life, are also known to exist. Sometimes gene alteration maintains high-level expressions of a gene resulting in, for example, gigantism. In other words, when a gene undergoes a certain alteration, only after experimental results are obtained can it be deduced whether the alteration results in a physiologically significant change, and if so, whether the change has a positive or negative impact.

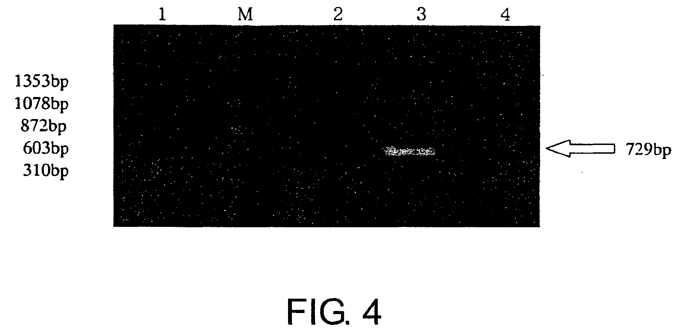

What biochemical changes does the 11-base deletion in exon 14 of the Mx1 gene discovered in the present invention bring on the propagation of RNA viruses? The Mx genes are established anti-influenza genes, and therefore, swine Mx1 can be expected to have anti-influenza activity as well. However, considering that the gene has as many as 14 exons in total, it is not easy to predict that an 11-base deletion would alter the ability to suppress influenza virus propagation. The present inventors found a pig carrying a three-base deletion in exon 13, and performed gene introduction and infection experiments (Fig. 4: 3T3-3 has retained the ability to suppress the propagation of an influenza virus). However, it was very difficult to predict the impact of each deletion.

Farm studies conducted by the present inventors revealed that the Landrace breed had the highest percentage of pigs with the homozygous 11-base deletion (14%), whereas in the other breeds, the homozygous deletion was rare and sometimes not found at all. On the other hand, the percentage of pigs with the heterozygous 11-base deletion in Landrace was very high (57%); 16% in Berkshire; 14% in Duroc; and 47% in Hampshire.

Even for those skilled in the art, it would be difficult to predict from such findings the kind of impact that the 11-base deletion in the last exon of the Mx 1 gene may have on the suppression of RNA virus propagation. The data on the gene nucleotide sequence and farm studies alone may lead to the assumption that these two types of Mx1 deletions do not have any impact. Alternatively, an assumption that the deletion is advantageous to survival cannot be denied based on the above-described data alone, although it is contrary to the findings of the present invention.

The finding that the Mx1 gene with the 11-base deletion can barely suppress influenza virus propagation, and the finding that the 3-base deletion in exon 13 does not result in the loss of the ability to suppress virus propagation, were first revealed by the present invention, which describes experimental results obtained by introducing the swine Mx1 gene into murine cells and infecting the transformed cells with an influenza virus.

The Mx1 gene product, Mx1 protein, functions in cooperation with other proteins within living cells (cytoplasm). To study how well (or how bad) the identified 11-base deletant can suppress viral propagation compared with the normal gene, the normal (wild-type) gene or the gene with the 11-base deletion was introduced into cells (murine 3T3 cells in the experiments described herein) that do not have viral propagation-suppressing abilities. The cells were cultured, and the swine Mx1 gene expressions were confirmed. The cells were then infected with an influenza virus, and the amounts of virus amplified in the cells were determined.

This series of experiments require advanced knowledge and technology associated with genome studies, and may have been difficult to carry out by ordinary research.

The present invention relates to methods for determining genetic resistance of pigs to diseases caused by RNA viruses. More specifically, the present invention provides:
[1] a method for determining a pig's resistance to an RNA virus, in particular an influenza virus or the virus that causes PRRS (resistance to a disease caused by an RNA virus), wherein the method comprises the step of detecting an 11-bp deletion in a swine Mx1 gene exon, wherein the deletion is from positions 2064 to 2074 in the nucleotide sequence of SEQ ID NO: 1;
[2] the method according to [1], comprising the steps of:
   (a) preparing a DNA sample from a subject pig;
   (b) amplifying a DNA that is a swine Mx1 gene exon and comprises the nucleotide sequence from positions 2064 to 2074 in the nucleotide sequence of SEQ ID NO: 1; and
   (c) determining the nucleotide sequence of the amplified DNA;
[3] the method according to [1], comprising the steps of:
   (a) preparing a DNA sample from a subject pig;
   (b) digesting the prepared DNA with a restriction enzyme;
   (c) separating DNA fragments based on their size; and
   (d) comparing the sizes of detected DNA fragments with that of a control;
[4] the method according to [1], comprising the steps of:
   (a) preparing a DNA sample from a subject pig;
   (b) amplifying a DNA that is a swine Mx1 gene exon and comprises the nucleotide sequence from positions 2064 to 2074 in the nucleotide sequence of SEQ ID NO: 1;
   (c) digesting the amplified DNA with a restriction enzyme;
   (d) separating DNA fragments based on their size; and
   (e) comparing the sizes of detected DNA fragments with that of a control;
[5] the method according to [1], comprising the steps of:
   (a) preparing a DNA sample from a subject pig;
   (b) amplifying a DNA that is a swine Mx1 gene exon and comprises the nucleotide sequence from positions 2064 to 2074 in the nucleotide sequence of SEQ ID NO: 1;
   (c) dissociating the amplified DNA into single strands;
   (d) separating the dissociated single-stranded DNAs on a non-denaturing gel; and
   (e) comparing the gel mobility of the fractionated single-stranded DNAs with that of a control;
[6] the method according to [1], comprising the steps of:
   (a) preparing a DNA sample from a subject pig;
   (b) amplifying a DNA that is a swine Mx1 gene exon and comprises the nucleotide sequence from positions 2064 to 2074 in the nucleotide sequence of SEQ ID NO: 1;
   (c) determining the molecular weight of the DNA amplified in step (b) by mass spectrometry; and
   (d) comparing the molecular weight determined in step (c) with that of a control;
[7] the method according to [1], comprising the steps of:
   (a) preparing a DNA sample from a subject pig;
   (b) amplifying a DNA that is a swine Mx1 gene exon and comprises the nucleotide sequence from positions 2064 to 2074 in the nucleotide sequence of SEQ ID NO: 1;
   (c) preparing a substrate with an immobilized nucleotide probe;
   (d) contacting the DNA prepared in step (b) with the substrate prepared in step (c);
   (e) determining the intensity of hybridization between the DNA and the nucleotide probe immobilized on the substrate; and
   (f) comparing the intensity determined in step (e) with that of a control;
[8] the method according to [1], comprising the steps of:
   (a) preparing a protein sample from a subject pig; and
   (b) determining the amount of a mutant swine Mx1 protein in the protein sample, wherein said mutant swine Mx 1 protein is encoded by a nucleotide sequence that is a swine Mx 1 gene exon in which the 11-bp nucleotide sequence from positions 2064 to 2074 in SEQ ID NO: 1 has been deleted;
[9] the method according to any one of [1] to [8], further comprising the step of determining that a subject pig is susceptible to an RNA virus when the 11-base deletion defined above is detected or the subject pig is resistant to the RNA virus when the deletion is not detectable;
[10] the method according to any one of [1] to [9], wherein the RNA virus is an influenza virus or the causative virus of PRRS;
[11] an oligonucleotide to be used as a PCR primer in the method according to any one of [1] to [10], wherein the oligonucleotide is used to amplify a DNA region that is a swine Mx1 gene exon and comprises the nucleotide sequence from positions 2064 to 2074 in the nucleotide sequence of SEQ ID NO: 1;
[12] an oligonucleotide comprising at least 15 nucleotides, and hybridizing to a DNA region that is a swine Mx1 gene exon and comprises the nucleotide sequence from positions 2064 to 2074 in the nucleotide sequence of SEQ ID NO: 1, or a DNA region that is a swine Mx1 gene exon and comprises a nucleotide sequence in which the nucleotide sequence from positions 2064 to 2074 has been deleted;
[13] an antibody recognizing a mutant swine Mx1 protein encoded by the nucleotide sequence of a swine Mx1 gene exon in which the nucleotide sequence from positions 2064 to 2074 in SEQ ID NO: 1 has been deleted;
[14] a test reagent for determining a pig's resistance to an RNA virus (resistance to a disease caused by an RNA virus), wherein the reagent comprises the oligonucleotide according to [11] or [12], or the antibody according to [13]; and
[15] the test reagent according to [14], wherein the RNA virus is an influenza virus or the causative virus of PRRS.

The present invention provides methods for determining pig resistance to an RNA virus, comprising the step of detecting an 11-bp deletion in an exon of the swine Mx 1 gene, wherein the deletion is from position 2064 to position 2074 in the nucleotide sequence of SEQ ID NO: 1.

Herein, the term "resistance to an RNA virus" refers to, for example, "resistance to a disease caused by an RNA virus".

Herein, the term "RNA virus" refers to, for example, influenza viruses and corona virus that causes PRRS (porcine reproductive and respiratory syndrome).

Influenza viruses and the causative virus of PRRS are similar to each other, both of which being either spherical or pleiomorphic virions in appearance. In addition, both viruses comprise a single-stranded linear RNA genome of 10-30 Kb in size, and the viral proteins are synthesized in the cytoplasm of their host cells. Mx1 protein inhibits the reverse transcription process in the cytoplasm, in which viral RNA is transcribed into host DNA and the transcribed information is again transcribed into RNA. Therefore, the present invention can also be applied to the causative virus of PRRS whose genome size is similar to that of influenza viruses. In the present invention, there is no limitation as to the type of RNA virus, but the RNA virus is preferably an influenza virus or the causative virus of PRRS.

The nucleotide sequence of the last exon of the Mx1 gene described above is shown in SEQ ID NO: 1, and the amino acid sequence of a protein encoded by the nucleotide sequence is shown in SEQ ID NO: 2. Those skilled in the art can readily obtain this sequence information using the PubMed Accession No. M65087.

The "deleted" 11-bp of the present invention has the sequence of 5'-gg cgc cgg ctc-3' in the last exon of the Mx1 gene, and corresponds to the nucleotide region from positions 2064 to 2074 in SEQ ID NO: 1. Even with slight nucleotide variations (nucleotide substitution, deletion, insertion, etc.) in a DNA sequence information equivalent to the 11-bp of the Mx1 gene disclosed in public databases, those skilled in the art can readily identify the region of the Mx 1 gene to which the "11-bp deletion" of the present invention corresponds based on the information disclosed herein. The "11-bp deletion" of the present invention includes the above-described "deletions corresponding to 11-bp deletions" which can be readily found by those skilled in the art.

Herein, the "11-bp deletion" is not restricted to the state in which only the 11-bp region is deleted. Specifically, the "11-bp deletion" of the present invention includes cases wherein DNA regions in addition to the 11-bp region have been deleted, for example, deletions comprising the 11-bp DNA and several base pairs adjacent to it.

In the present invention, a subject pig in which a heterozygous "11-bp deletion" as described above has been detected in the last exon of its Mx1 gene, is judged to have an Mx1 protein with no ability to suppress RNA viruses (for example, influenza viruses and the causative virus of PRRS). Alternatively, the cells of a subject pig carrying a homozygous "11-bp deletion" as described above are judged to have only an Mx1 protein with no ability to suppress RNA viruses (for example, influenza viruses and the causative virus of PRRS), and thus the subject pig is judged to exhibit high susceptibility to RNA viruses (for example, influenza viruses and the causative virus of PRRS). A subject pig is judged to be resistant to RNA viruses (for example, influenza viruses and the causative virus of PRRS) if the "11-bp deletion" described above is not detected.

In the present invention, there are no limitations on the methods (means) for detecting the above-described "11-bp deletion" in the last exon of the swine Mx1 gene, as long as the methods (means) can detect a "deletion" as defined above. For example, such a method may comprise the step of directly determining the nucleotide sequence of the last exon containing the above-defined deletion portion in the Mx1 gene of a subject pig.

In this method, first, a DNA sample is prepared from a subject pig. The DNA sample can be prepared from, for example, chromosomal DNA extracted from organs, tissues, cells, blood, oral mucosal membranes, skin, or hair of the subject pig, and alternatively cDNA or mRNA that contains no introns.

Next step in the method involves isolating a DNA that is an exon of the swine Mx1 gene and comprises the nucleotide sequence from positions 2064 to 2074 in the nucleotide sequence of SEQ ID NO: 1. The DNA can be isolated by PCR or such using chromosomal DNA or RNA as a template and using primers that hybridize to the last exon of the swine Mx1 gene. Then, the nucleotide sequence of the isolated DNA is determined. The nucleotide sequence of the isolated DNA can be determined by using methods known to those skilled in the art.

In the next step of the method, the determined nucleotide sequence of the DNA is compared to that of a control. In this method, the control refers to the sequence of the normal (wild type) swine Mx1 gene (for example, SEQ ID NO: 1).

Besides the methods which directly determine the nucleotide sequence of DNA derived from a subject pig as described above, different possible methods to determine the above-described "deletion" can also be used for the determination method of the present invention.

For example, detection of the above-described "deletion" of the present invention can also be achieved using the method described below.

First, a DNA sample is prepared from a subject pig. Then, the prepared DNA sample is digested with restriction enzymes. In the next step, DNA fragments are fractionated based on their size. The sizes of the detected DNA fragments are then compared with that of the control. In another embodiment, first, a DNA sample is prepared from a subject pig, followed by the amplification of a DNA that is an exon of the swine Mx1 gene and comprises the nucleotide sequence from positions 2064 to 2074 in the nucleotide sequence of SEQ ID NO: 1. The amplified DNA is then digested with restriction enzymes. In the next step, DNA fragments are fractionated based on their size. The sizes of the detected DNA fragments are then compared with that of the control.

Such methods include, for example, methods using Restriction Fragment Length Polymorphism (RFLP) and PCR-RFLP. Specifically, when a restriction enzyme recognition site is mutated (deleted), the fragment sizes yielded by restriction enzyme treatments are different from the control. The region containing the mutation is amplified by PCR, and digested with different restriction enzymes. The mutation can be detected based on the difference of band mobility in electrophoresis. Alternatively, chromosomal DNA is digested with these restriction enzymes and electrophoresed. The mutation can be detected by Southern blotting using a probe DNA of the present invention. Such restriction enzymes can be appropriately selected depending on the mutation. In this method, instead of genomic DNA, cDNA prepared from the RNA of a subject pig using reverse transcriptase can also be used. The cDNA can be directly digested with restriction enzymes and then analyzed by Southern blotting. Alternatively, the DNA which is an exon of the swine Mx1 gene and comprises the nucleotide sequence from positions 2064 to 2074 in the nucleotide sequence of SEQ ID NO: 1, is amplified by PCR using the cDNA as a template, subjected to restriction enzyme treatments, and examined for differences in band mobility.

In an alternative method, first, a DNA sample is prepared from a subject pig. Then, the DNA which is an exon of the swine Mx1 gene and comprises the nucleotide sequence from positions 2064 to 2074 in the nucleotide sequence of SEQ ID NO: 1, is amplified. Then, the amplified DNA is dissociated into single-stranded DNAs. In the next step, the dissociated single-strand DNAs are fractionated on a non-denaturing gel. The electrophoretic mobility of the fractionated single-stranded DNAs is compared to that of the control.

This method is exemplified by PCR-SSCP (single-strand conformation polymorphism; Cloning and polymerase chain reaction-single-strand conformation polymorphism analysis of anonymous Alu repeats on chromosome 11. Genomics. 1992 Jan 1, 12(1), 139-146; Detection of p53 gene mutations in human brain tumors by single-strand conformation polymorphism analysis of polymerase chain reaction products. Oncogene. 1991 Aug 1, 6(8), 1313-1318; Multiple fluorescence-based PCR-SSCP analysis with postlabeling. PCR Methods Appl. 1995 Apr 1, 4(5), 275-282). This method has advantages such as relatively simple operation and small amounts of sample required, and is thus particularly suitable for screening large quantities of DNA samples. The principle of the method is as follows. When a double-stranded DNA fragment is dissociated into single strands, each strand forms its own conformation depending on the nucleotide sequence. The dissociated DNA stands are electrophoresed on a polyacrylamide gel containing no denaturant. The complementary single-stranded DNAs which are identical in length move to different locations because of their conformational differences. Even with a single nucleotide substitution, the single-stranded DNA conformation is altered, resulting in different electrophoretic mobility in polyacrylamide gel electrophoresis. Thus, mutations such as deletions in a DNA fragment can be detected by determining the electrophoretic mobility.

Specifically, first, the DNA which is an exon of the swine Mx1 gene and comprises the nucleotide sequence from positions 2064 to 2074 in the nucleotide sequence of SEQ ID NO: 1, is amplified by PCR or the like. Generally, a preferred size of the PCR-amplified region ranges from about 200 to 400 bp. Those skilled in the art can appropriately select the PCR conditions and such. The amplified DNA products can be labeled by using PCR primers labeled with an isotope such as ³²P, fluorescent dye, biotin, or such. Alternatively, the amplified DNA products can be labeled using a PCR solution containing substrate nucleotides labeled with an isotope such as ³²P, fluorescent dye, biotin, or such. Alternatively, the amplified DNA fragments can be labeled by using the Klenow enzyme or such to add substrate nucleotides labeled with an isotope such as ³²P, fluorescent dye, biotin, or such after PCR is completed. The labeled DNA fragments are denatured by heating, and then electrophoresed on a polyacrylamide gel containing no denaturant such as urea. The conditions for separating the DNA fragments can be improved by adding an adequate amount of glycerol (about 5-10%) to the polyacrylamide gel. The electrophoresis conditions are appropriately selected depending on the properties of the respective DNA fragments. Typically, electrophoresis is carried out at room temperature (20-25°C). When the separation is insufficient, a temperature that gives optimal mobility is selected from 4 to 30°C. Following electrophoresis, the DNA fragments are analyzed for their mobility by autoradiography using X-ray film, fluorescence scanner, or the like. Bands that have differential mobility are identified, excised directly from the gel, amplified again by PCR and then sequenced directly. The presence of a mutation can be confirmed based on the sequence. Unlabeled DNA can also be used and detected as bands by staining the gel using ethidium bromide, sliver staining, or the like, after electrophoresis is completed.

In an alternative method, first, a DNA that is an exon of the swine Mx1 gene and comprises the nucleotide sequence from positions 2064 to 2074 in the nucleotide sequence of SEQ ID NO: 1 is prepared from a subject pig, and a substrate is prepared to which nucleotide probes that hybridize to the DNA have been immobilized. Then, the DNA is contacted with the substrate. The "deletion" described above is detected by detecting the DNAs hybridized to the nucleotide probes immobilized on the substrate.

Such a method is exemplified by DNA array. Methods known to those skilled in the art can be used to prepare from a subject pig, a DNA sample that is an exon of the swine Mx1 gene and comprises the nucleotide sequence from positions 2064 to 2074 in the nucleotide sequence of SEQ ID NO: 1. In a preferred embodiment, the DNA sample may be prepared from, for example, chromosomal DNA extracted from tissues or cells of skin, oral mucosal membranes, blood, or the like of a subject pig. The DNA sample to be used in the method described above can be prepared from chromosomal DNA as follows. A DNA that is a swine Mx1 gene exon and comprises the nucleotide sequence from positions 2064 to 2074 in the nucleotide sequence of SEQ ID NO: 1, is prepared by, for example, PCR using chromosomal DNA as a template, and primers that hybridize to the DNA that is a swine Mx 1 gene exon and comprises the nucleotide sequence from positions 2064 to 2074 in the nucleotide sequence of SEQ ID NO: 1. The prepared DNA sample can be labeled for the convenience of detection using methods known to those skilled in the art, as necessary.

Herein, the term, "substrate" refers to a plate-shaped material onto which nucleotides can be immobilized. Herein, the term "nucleotide" refers to both oligonucleotides and polynucleotides. There are no limitations on the substrate of the present invention, as long as nucleotides can be immobilized on it. Substrates routinely used in the DNA array technology can be used suitably in the present invention.

Typical DNA array is composed of thousands of nucleotides densely printed on a substrate. Typically, these DNAs are printed on the surface of a non-porous substrate. The surface of a typical substrate is made of glass. However, it may be made of a porous membrane, for example, nitrocellulose membrane.

Methods of immobilizing nucleotides (array) used in the present invention include the oligonucleotide-based array method developed by Affymetrix. Oligonucleotides used in the oligonucleotide-based array are typically synthesized *in situ*. Any of the known methods of synthesizing oligonucleotides *in situ* based on, for example, the photolithography technology (Affymetrix) or ink-jet printing technology for immobilization of substances (Rosetta Inpharmatics), can be used to prepare the substrates of the present invention.

There are no limitations on the nucleotide probe to be immobilized onto a substrate, as long as it can detect the above-described "deletion". Specifically, the probe includes, for example, probes hybridizing specifically to a DNA that is a wild-type swine Mx1 gene exon and comprises the nucleotide sequence from positions 2064 to 2074 in the nucleotide sequence of SEQ ID NO: 1, or a DNA that is a swine Mx1 gene exon in which the nucleotide sequence from positions 2064 to 2074 of the nucleotide sequence in SEQ ID NO: 1 have been deleted. As long as specific hybridization is possible, the nucleotide probe needs not be completely complementary to the DNA to be detected which is a wild-type swine Mx1 gene exon and comprises the nucleotide sequence from positions 2064-2074 in the nucleotide sequence of SEQ ID NO: 1, or the DNA which is a swine Mx1 gene exon in which the nucleotide sequence from positions 2064 to 2074 of the nucleotide sequence in SEQ ID NO: 1 have been deleted.

In the present invention, when oligonucleotides are immobilized, the length of a nucleotide probe to be bound to a substrate is typically 10-100 bases, preferably 10-50 bases, more preferably 15-25 bases.

Next step in the present invention involves contacting a cDNA sample with the substrate. In this step, a DNA sample is hybridized to the above-described nucleotide probes. Hybridization solution and conditions vary depending on factors such as the length of the nucleotide probes to be immobilized onto the substrate; however, the hybridization can generally be carried out using methods known to those skilled in the art.

Next step in the present invention involves determining whether the DNA sample is hybridized to the nucleotide probes immobilized on the substrate, and the intensity of the hybridization. This detection can be achieved by, for example, reading fluorescence signals using a scanner or such. In DNA array technology, DNA immobilized on a glass slide is generally referred to as the "probe", while labeled DNA in the solution is referred to as the "target". Therefore, the above-described nucleotides immobilized on the substrate are referred to as "nucleotide probes" herein.

In addition to the methods described above, the allele specific oligonucleotide (ASO) hybridization method can be used to detect deletions at a particular position. An oligonucleotide comprising a nucleotide sequence with potential mutations (deletions) is prepared, and hybridized with a sample DNA. Mutations in the DNA would result in a decrease in the efficiency of hybrid formation. The degree of hybridization can be determined by, for example, Southern blotting or methods using quenching resulting from the intercalation of a specific fluorescent reagent into the gaps of the hybrid. The ribonuclease A mismatch cleavage method can also be used. Specifically, a DNA that is a swine Mx1 gene exon and comprises the nucleotide sequence from positions 2064 to 2074 in the nucleotide sequence of SEQ ID NO: 1, is amplified by PCR or such. The amplified DNA is hybridized with a labeled RNA prepared from, for example, a cDNA of the last exon of the Mx1 gene incorporated into a plasmid vector. The hybrid has a partially single-stranded structure where the mutation is present. The single-stranded portion is cleaved by ribonuclease A, and detected using autoradiography or such. The mutation can be detected using the method described above.

In an alternative method, first, DNA is prepared from a subject pig, and then a DNA which is a swine Mx1 gene exon and comprises the nucleotide sequence from positions 2064 to 2074 in the nucleotide sequence of SEQ ID NO: 1, is amplified. The amplified DNA is then analyzed by mass spectrometry to determine its molecular weight. The determined molecular weight is compared to that of a control. The method includes, for example, MALDI-TOF MS (Trends Biotechnol, 18, 77-84 (2000)).

In another embodiment of the present invention, the above-described detection method is a detection method using the expression products of the last exon of the swine Mx1 gene as an indicator. Herein, the term "expression" refers to both transcription and translation. Accordingly, the "expression products" include both mRNA and proteins.

The present invention provides a method of assessing pigs that have resistance against RNA viruses (for example, influenza viruses and the causative virus of PRRS), wherein the method comprises detecting expression products of the last exon of the swine Mx1 gene. In a preferred embodiment of the method, first, a protein sample is prepared from a subject pig, and then the amount of a mutant swine Mx1 protein contained in the sample is determined, wherein the mutant protein is encoded by the nucleotide sequence that is a swine Mx1 gene exon in which the 11-bp segment from positions 2064 to 2074 in SEQ ID NO: 1 has been deleted.

Such a method includes SDS-polyacrylamide gel electrophoresis; and Western blotting, dot blotting, immunoprecipitation, enzyme-linked immunosorbent assay (ELISA), and immunofluorescence method which use an antibody against a mutant swine Mx1 protein encoded by a nucleotide sequence that is a swine Mx1 gene exon in which the 11-bp segment from positions 2064 to 2074 in SEQ ID NO: 1 has been deleted.

A subject pig is determined to be susceptible to RNA viruses (for example, influenza viruses and the causative virus of PRRS), when a mutant swine Mx 1 protein encoded by the nucleotide sequence that is a swine Mx1 gene exon in which the 11-bp segment from positions 2064 to 2074 in SEQ ID NO: 1 has been deleted, is detected using the method described above. In contrast, when the above-described mutant protein is not detected, a subject pig is determined to be resistant to RNA viruses (for example, influenza viruses and the causative virus of PRRS).

The detection method is not restricted to the various detection methods described above.

The present invention also provides test reagents to be used in the methods of the present invention for assessing pigs that have resistance to RNA viruses (for example, influenza viruses and the causative virus of PRRS).

In an embodiment, the reagent is a test reagent comprising an oligonucleotide of at least 15 nucleotides that hybridizes to a DNA region, which is a swine Mx1 gene exon and comprises the nucleotide sequence from positions 2064 to 2074 in the nucleotide sequence of SEQ ID NO: 1, or a DNA region which is a swine Mx1 gene exon and comprises a nucleotide sequence containing a deletion of positions 2064 to 2074.

The oligonucleotide hybridizes specifically to a DNA region that is a swine Mx1 gene exon and comprises the nucleotide sequence from positions 2064 to 2074 in the nucleotide sequence of SEQ ID NO: 1, or a DNA region that is a swine Mx1 gene exon and comprises a nucleotide sequence containing a deletion at positions 2064 to 2074. Herein, the phrase "hybridizes specifically" means that there is no significant cross-hybridization between the oligonucleotide and DNAs encoding other proteins under standard hybridization conditions or preferably under stringent hybridization conditions (for example, the conditions described in Sambrook *et al*., "Molecular Cloning", 2nd Ed., Cold Spring Harbor Laboratory Press, New York, USA, 1989). It is not necessary for an oligonucleotide to be completely complementary to the above-described nucleotide sequence to be detected, as long as it can hybridize specifically.

The oligonucleotide can be used as a probe or primer in the detection methods of the present invention. When the oligonucleotide is used as a primer, its length is typically 15-100 bp, and preferably 17-30 bp. There are no limitations on the primer, as long as it allows the amplification of a DNA region that is a swine Mx1 gene exon and comprises the nucleotide sequence from positions 2064 to 2074 in the nucleotide sequence of SEQ ID NO: 1. Such primers include, though not limited to, for example, oligonucleotides comprising the sequence of SEQ ID NO: 9 or 10 described below in the Example.

Alternatively, when the oligonucleotide of the present invention is used as a probe, there are no limitations on the probe, as long as it hybridizes specifically to at least a portion of a DNA region that is a swine Mx 1 gene exon and comprises the nucleotide sequence from positions 2064 to 2074 in the nucleotide sequence of SEQ ID NO: 1, or a portion of a DNA region that is a swine Mx1 gene exon and comprises a nucleotide sequence containing a deletion of positions 2064 to 2074. The probe can be a synthetic oligonucleotide, and is typically at least 15 bp long.

The oligonucleotides of the present invention can be prepared by, for example, using a commercially available oligonucleotide synthesizer. The probes can also be prepared as double-stranded DNA fragments from restriction enzyme treatments or the like.

The oligonucleotides of the present invention can be suitably used as probes after proper labeling. The labeling method includes, for example, labeling methods that phosphorylate the 5' end of an oligonucleotide with ³²P using the T4 polynucleotide kinase; methods such as the random primer method, in which substrate nucleotides labeled with an isotope such as ³²P, fluorescent dye, biotin, or such are incorporated into DNA, by using a primer such as random hexamer oligonucleotide or the like, and a DNA polymerase such as the Klenow enzyme.

In another embodiment, the test reagent of the present invention is a reagent comprising an antibody against a mutant swine Mx1 protein encoded by the nucleotide sequence that is a swine Mx1 gene exon in which the 11-bp segment from positions 2064 to 2074 in SEQ ID NO: 1 has been deleted. There are no limitations on the antibodies of the present invention, as long as they can be applied to the methods of this invention. Such an antibody includes, for example, polyclonal and monoclonal antibodies. The antibody may be labeled as necessary.

The antibodies against a mutant swine Mx1 protein encoded by the nucleotide sequence that is a swine Mx1 gene exon in which the 11-bp segment from positions 2064 to 2074 in SEQ ID NO: 1 has been deleted, can be prepared by methods known to those skilled in the art. Polyclonal antibodies can be prepared by the procedure described below. Small animals such as rabbits are immunized with a mutant swine Mx 1 protein, or a recombinant mutant swine Mx 1 protein or a partial peptide thereof, obtained by expressing its GST fusion protein in microorganisms such as E. coli. Sera collected from the animals are purified, for example, by using ammonium sulfate precipitation, protein A column, protein G column, DEAE ion exchange chromatography, or affinity column coupled with the mutant swine Mx1 protein or synthetic peptide. Monoclonal antibodies can be prepared by, for example, immunizing a small animal such as mouse with a mutant swine Mx1 protein or a partial peptide thereof, extracting and gently crushing the mouse spleen to isolate cells. The cells obtained are fused with mouse myeloma cells using reagents such as polyethylene glycol. From these fused cells (hybridoma), clones producing antibodies that bind to the mutant swine Mx1 protein are selected. Next, the resulting hybridomas are transplanted into the peritoneal cavity of a mouse, from which ascites is collected. The monoclonal antibody prepared is purified by, for example, using ammonium sulfate precipitation, protein A column, protein G column, DEAE ion exchange chromatography, affinity column coupled with the mutant swine Mx1 protein or synthetic peptide.

The test reagent described above may contain, in addition to an oligonucleotide or antibody as the active ingredient, sterilized water, physiological saline, a vegetable oil, a detergent, a lipid, a solubilizing agent, a buffer, a protein stabilizer (BSA, gelatin, etc.), a preservative, or such, as necessary.

### Brief Description of the Drawings

Fig. 1 is a photograph showing a three-way crossbred pig used for fattening/production.
Fig. 2 is a graph showing the ability of each Mx 1 genotype to suppress viral propagation at an MOI of 1.
Fig. 3 is a graph showing the ability of each Mx1 genotype to suppress viral propagation at an MOI of 10.
Fig. 4 is a photograph that compares the Mx1 expression levels in the respective transformed cells of 3T3, 3T3-0, 3T3-3, and 3T3-11, based on the results obtained using RT-PCR.
Fig. 5 shows an "11-bp deletion" region in the last exon of the swine Mx1 gene, and nucleotide sequences of a primer pair to be used for amplifying the region.
Fig. 6 is a photograph showing the detection of an 11-base deletion in the last exon of the swine Mx 1 gene using PCR amplification.

### Best Mode for Carrying Out the Invention

Herein below, the present invention will be specifically described using Examples, however, it is not to be construed as being limited thereto.

It was examined whether or not the Mx1 gene mutations identified in the swine breeds Meishan and Landrace had an impact on the function of the expressed protein.

Specifically, whether or not the Mx1 gene mutations affect the defense function against infection was examined by carrying out experiments to infect cells harboring a forced-expressed normal or mutant Mx1 gene with an influenza virus.

### [Example 1] cDNA cloning of normal and mutant Mx1 genes from Meishan and Landrace pigs

5 ml of EDTA blood was collected from each of the 39 Meishan pigs and 36 Landrace pigs. The blood samples were diluted twice with RPMI 1640 (GIBCO), overlaid on Ficoll-Paque (Pharmacia Biotech), and then centrifuged at 400x g for 40 minutes. The layer containing lymphocytes was collected, and the cells were washed three times with phosphate buffered saline. The prepared lymphocytes were treated with DNAZol Reagent (GIBCO) for DNA purification. PCR amplification was performed using the purified DNAs as a templates at conditions of 5 minutes at 94°C; 40 cycles of 30 seconds at 94°C, 30 seconds at 55°C, and 1 minute at 72°C ; and 5 minutes at 72°C. Exon 13 was amplified from the DNA from Meishan pigs using a primer pair: 5'-CTGAAAGATCTCGGCTATGGAGG-3'/SEQ ID NO:3; and 5'-AAGAAGCTGAGACGTCGATCCGGCT-3'/SEQ ID NO: 4. The last exon was amplified from the DNA from Landrace pigs using the primer pair 5'-AAGCGCATCCAGCCACATC-3'/SEQ ID NO: 5; and 5'-AAGACATTGGGCGTGAAAGG-3'/SEQ ID NO: 6. The presence of the mutation in exon 13 was determined by direct sequencing. The presence of the mutation in the last exon was determined by examining the RFLP after Nal I treatment at 37°C for 2 hours.

20 ml of EDTA blood was collected from animals that had been identified to carry a normal or mutant Mx1 gene. Lymphocytes were prepared from the blood, and suspended in RPMI 1640 containing 10% fetal bovine serum (FBS). The cell suspensions were incubated in a CO₂ incubator at 37°C overnight. The cells were treated with 500 U human IFNα (Calbiochem) for three hours. RNAs were purified from the treated lymphocytes. The Mx1 gene was amplified by RT-PCR using the RNAs as templates and the primer pair attB1mxF (5'-GGGGACAAGTTTGTACAAAAAAGCAGGCTGTCACAGCGTCAAAGAAAAGGAAG-3'/SEQ ID NO: 7) and attB2mxR (5'-GGGGACCACTTTGTACAAGAAAGCTGGGTCCTTCTATGATGCTATGCGG-3'/SEQ ID NO: 8). The PCR products were combined with the pDONR201 vector from Gateway Cloning Technology (GIBCO). The mixtures were treated with BP Clonase (GIBCO) at 25°C for one hour. The BP reaction solution was combined with DH5α competent cells for transformation in a routine fashion, and selection of transformants was performed using LB plates containing 50 µg/ml kanamycin. The nucleotide sequence of the insert in each clone was determined by direct sequencing of PCR products obtained by colony PCR using attB1mx-F and attB2mx-R (2 minutes at 94°C ; 30 cycles of 30 seconds at 94°C , 30 seconds at 64°C , and 3 minutes at 72°C ; 5 minutes at 72°C). Normal Mx1 cDNA clone is referred to as pEntmx1, Meishan cDNA clone is referred to as pEntmx1-3, and Landrace cDNA clone is referred to as pEntmx1-11.

### [Example 2] Construction of Mx1 gene expression vectors

Each of the recombinant clones, pEntmx1, pEntmx1-3, and pEntmx1-11, was inoculated into 15 ml of LB, cultured while shaking at 37°C overnight, and the plasmid DNAs were purified by the alkaline isolation method. The plasmid DNAs were combined with pDEST12.2 (GIBCO). After treatment with LR Clonase (GIBCO) at 25°C for 60 minutes, the DNAs were transformed into DH5α competent cells. The selection of transformants was performed using LB containing 100 µg/m ampicillin. Plasmid DNAs were purified from the respective recombinant clones, which are designated as pExmx1, pExmx1-3, and pExmx1-11. The nucleotide sequence of the insert in each clone was determined by direct sequencing of PCR products obtained by colony PCR using attB1mx-F and attBmx-R.

### [Example 3] Preparation of Mx 1 gene transformants

NIH3T3 cells were passaged and maintained using Dulbecco's modified MEM (DMEM, GIBCO) containing 7% FBS. 5 µg of pExmx1, pExmx1-3, and pExmx1-11 was each combined with 0.4 ml of 3T3 cell suspension (4x 10⁶ cells), placed in 2-mm-thick cuvettes, and electroporated at 100 µF, 13 ohm, 200 v, and 1 kv/cm. After incubation for four days, the cells were cultured in DMEM containing 500 µg/ml G418 (Geneticin; GIBCO) for the selection of transformants. The pExmx1 transformant is designated as 3T3-0, pExmx1-3 transformant as 3T3-3, and pExmx1-11 transformant as 3T3-11.

### [Example 4] Preparation of influenza virus particles

An influenza virus (Aichi; H2N2) was inoculated into the allantoic cavities of 11-day-old SPF eggs. The eggs were incubated at 37°C for two days, and then allowed to stand overnight at 4°C. Allantoic fluids were collected from the eggs, and then centrifuged at 3500 rpm for 20 minutes. Aliquots of the supernatant (seed virus solution) were stored at -80°C. The titer of seed virus was estimated to be 107.5 EID₅₀ (50% Egg-Infective Dose)/0.2 ml.

### [Example 5] Influenza virus infection experiments

Whether forced expression of each Mx 1 gene type has an impact on the viral propagation was examined by varying the virus dose used for infection.

3T3, 3T3-0, 3T3-3, and 3T3-11 cells were prepared at a cell density of 5x 10⁴ cells/ml. 1 ml of each cell suspension was added to the wells of a 24-well plate. The cells were incubated in a CO₂ incubator at 37°C for two days, and washed once with phosphate buffered saline (PBS). The seed virus solution was diluted 1:10 (corresponding to MOI (multiplicity of infection) =10) and 1:100 (corresponding to MOI =1) in PBS. The cells were inoculated with the diluted viral solutions, and incubated for adsorption at 37°C for one hour. Unadsorbed virus was removed by washing the cells twice with PBS. 2 ml of DMEM was added to the cells, and the cells were then incubated. Sampling was carried out at 0, 6, 12, 18, 24, 30, 36, 48, and 54 hours. The collected culture supernatants were centrifuged at 2500 rpm for 5 minutes. The supernatants were stored at -80°C until they were assayed for viral titer.

Viral titers in the supernatants collected over time were determined as described below. Serial 1:10 dilutions of each viral solution were prepared in PBS. 0.2 ml of each diluted viral solution was inoculated into the allantoic cavities of two 11-day-old eggs. After incubation for two days, the eggs were allowed to stand at 4°C overnight. The allantoic fluids were analyzed for hemagglutinating activity.

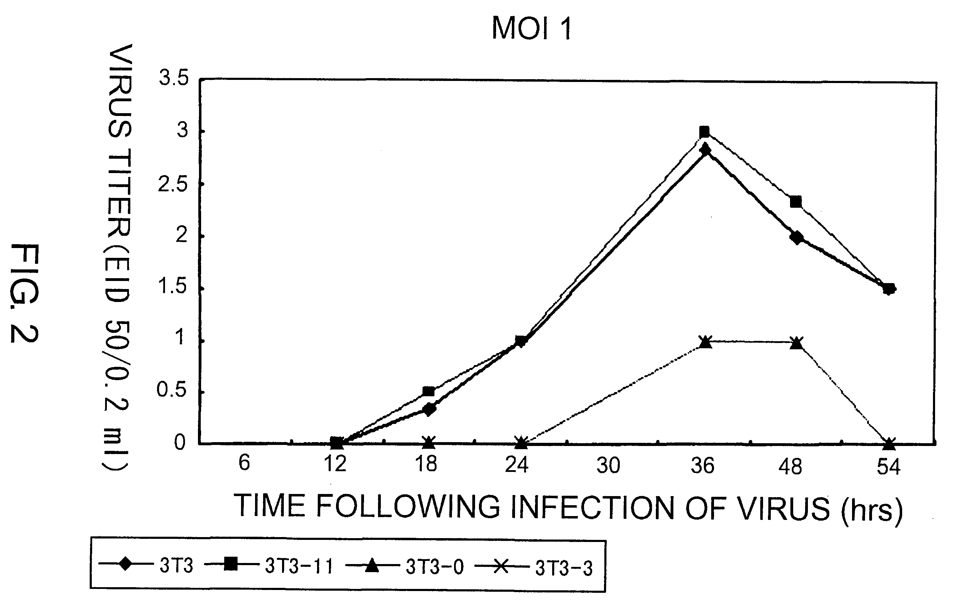

At MOI=1, the release of propagating virus into culture supernatants was recorded 18 hours after virus infection, and reached its peak of 2.8-3.0 EID₅₀/0.2 ml in 36 hours in the case of 3T3 and 3T3-11 cells. On the other hand, 3T3-0 and 3T3-3 cells have 1.0 EID₅₀/0.2 ml of propagating virus after 36-48 hours. However, no further increase in the viral propagation was recorded. Thus, the virus propagation was clearly different from that observed with the 3T3 and 3T3-11 cells (Fig. 2).

In the case of the 3T3 and 3T3-11 cells, at MOI=10, 0.3-0.5 EID₅₀/0.2 ml of infectious virus was detected in culture supernatants 12 hours after virus infection, and the viral titer reached its peak of 4.5-4.7 EID₅₀/0.2 ml in 36 hours. On the other hand, when 3T3-0 and 3T3-3 cells were used, the titer of infectious virus was 0.5 EID₅₀/0.2 ml after 24 hours, 2.7-2.8 EID₅₀/0.2 ml after 48 hours, and no further increase in the viral propagation was recorded after that. Thus, the viral propagation was evidently suppressed (Fig. 3).

Influenza virus is an RNA virus. Therefore, there is a possibility that the infection of 3T3 cells may result in the production of mouse IFNα, which affects viral propagation. Accordingly, another infection experiment was carried out, in which cells of each 3T3 cell type were infected with the virus at MOI=10, passaged and maintained in culture media containing anti-mouse IFNα for 12 days. Sampling was carried out after 36 hours. The viral titers in the culture supernatants were determined, and the results showed that the degree of viral propagation was comparable to that with cell groups that had not been treated with anti-mouse IFNα. This suggests that the factor affecting viral propagation is the introduced swine Mx 1 gene.

### [Example 6] Comparison of Mx1 expression levels in transformed cells

To examine whether the difference in viral propagation reflects the level of Mx1 expression in the respective types of cells, RT-PCR was performed using RNAs extracted from the respective types of cells used in the infection experiments.

3T3, 3T3-3, and 3T3-11 cells were prepared at a cell density of 2x 10⁶ cells/ml. RNAs were extracted and purified from the cells, and the Mx1 expression levels were determined by RT-PCR. The primer pair attBlmxF and attB2mxR was used. The PCR conditions were: 30 minutes at 55°C; 2 minutes at 94°C; 40 cycles of 15 seconds at 94°C, 30 seconds at 58°C, and 3 minutes at 68°C; 5 minutes at 72°C for extension. For external standard quantification, mouse G3PDH (TOYOBO) was amplified and used as a positive control for the expression level. A reaction solution to which distilled water was added instead of the template DNA was used as a negative control.

According to the results obtained, while the swine Mx 1 gene was not expressed in 3T3 cells (lane 1 in Fig. 4), the expression of the swine Mx1 gene was detectable in 3T3-0, 3T3-11, and 3T3-3 cells (lanes 3, 4 and 5 in Fig. 4), and slightly higher in 3T3-11 cells. The expression level of the internal standard mouse G3PDH suggested that the numbers of collected cells did not differ substantially among the respective cell types, and thus the levels of the Mx1 protein produced in 3T3-0, 3T3-11, and 3T3-3 cells were comparable to each other.

### [Example 7] Detection of an 11-base deletion in the last exon of swine Mx1 gene by PCR amplification

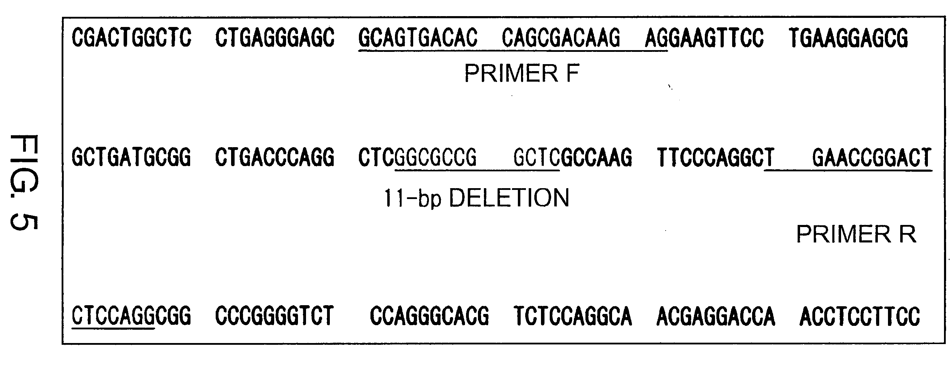

The following primer set was used in PCR for the subject pigs. The portion to be amplified was at positions 1981 to 2160 in the sequence of PubMed accession No. M65087 (Fig. 5).

Primer F: 5'- AGT GAC AGG AGC GAC AAG AG -3' (SEQ ID NO: 9)

Primer R: 5'- CCT GGA GAG TCC GGT TCA -3' (SEQ ID NO: 10)

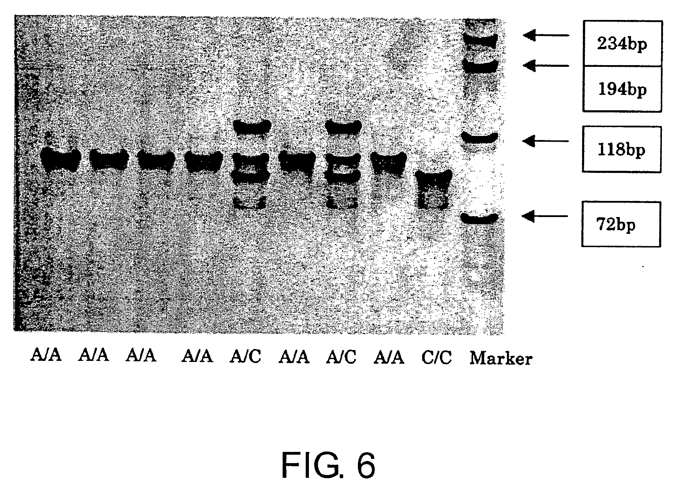

PCR conditions were: 1) 10 minutes at 94°C; 2) 30 seconds at 94°C, 30 seconds at 60°C, and 1 minute at 72°C; 3) 5 minutes at 72°C. Then, 2 µl of the PCR products was electrophoresed on a 6 % polyacrylamide gel at 200 V for 1 hour. The gel was sliver stained (Fig. 6). The wild type PCR product had a size of 105 bp. The 11-bp deletion product has a size of 94 bp. The size marker used was fX174/HaeIII.

As seen in Fig. 6, the 11-bp deletion could be detected by PCR using the above-described primer pair.

### Industrial Applicability

Among domestic pigs used worldwide, there are some that carry an Mx1 gene containing a 11-bp deletion which results in the loss of the ability to suppress viral propagation. Such animals are expected to have a serious defect in their defense ability during the early phase of an RNA virus invasion.

### [Simplified procedures for determining genetic disease resistance]

Animals carrying the wild-type gene which confers virus-suppressing ability can be selected from such pig populations, by collecting a small amount of DNA-containing tissues such as flesh and hair root, or blood, and using the extracted DNA to determine genotypes of the Mx1 gene, which is the subject of interest in the present invention. Alternatively, animals comprising undesired genotypes can be eliminated.

### [Improved pig production]

The level of genetic resistance to diseases caused by RNA viruses, including influenza viruses and the causative virus of PRRS, can be studied according to the present invention. Healthy animals which are more favorable for pig production can be selected based on the information. Furthermore, the incidence of respiratory diseases in piglets can be reduced, leading to increases in the survival and growth rates.

### [For human health]

Pig populations with impaired ability to suppress influenza virus propagation are susceptible to influenza infection. Unless all of the pigs die, the influenza virus will propagate and undergo genetic mutations, and in some cases evolve into new strains of influenza virus. Like the influenza virus that caused the Spanish influenza epidemic between 1917-1918, resulting in more than 40 million deaths around the world and more than 300,000 deaths in Japan alone, a new strain of influenza virus can cause enormous damage if it infects humans. By selecting pigs with a high ability to suppress influenza virus propagation according to the present invention, the propagation of an influenza virus can be suppressed in pig populations, thereby reducing the chance of the emergence of new influenza virus strains, and raising the possibility of eliminating one threat against humans.

## Claims

1. A method for determining a pig's resistance to an RNA virus, wherein the method comprises the step of detecting an 11-bp deletion in a swine Mx1 gene exon, wherein the deletion is from positions 2064 to 2074 in the nucleotide sequence of SEQ ID NO: 1.

2. The method according to claim 1, comprising the steps of:
(a) preparing a DNA sample from a subject pig;
(b) amplifying a DNA that is a swine Mx1 gene exon and comprises the nucleotide sequence from positions 2064 to 2074 in the nucleotide sequence of SEQ ID NO: 1; and
(c) determining the nucleotide sequence of the amplified DNA.

3. The method according to claim 1, comprising the steps of:
(a) preparing a DNA sample from a subject pig;
(b) digesting the prepared DNA with a restriction enzyme;
(c) separating DNA fragments based on their size; and
(d) comparing the sizes of detected DNA fragments with that of a control.

4. The method according to claim 1, comprising the steps of:
(a) preparing a DNA sample from a subject pig;
(b) amplifying a DNA that is a swine Mx1 gene exon and comprises the nucleotide sequence from positions 2064 to 2074 in the nucleotide sequence of SEQ ID NO: 1;
(c) digesting the amplified DNA with a restriction enzyme;
(d) separating DNA fragments based on their size; and
(e) comparing the sizes of detected DNA fragments with that of a control.

5. The method according to claim 1, comprising the steps of:
(a) preparing a DNA sample from a subject pig;
(b) amplifying a DNA that is a swine Mx1 gene exon and comprises the nucleotide sequence from positions 2064 to 2074 in the nucleotide sequence of SEQ ID NO: 1;
(c) dissociating the amplified DNA into single strands;
(d) separating the dissociated single-stranded DNAs on a non-denaturing gel; and
(e) comparing the gel mobility of the fractionated single-stranded DNAs with that of a control.

6. The method according to claim 1, comprising the steps of:
(a) preparing a DNA sample from a subject pig;
(b) amplifying a DNA that is a swine Mx1 gene exon and comprises the nucleotide sequence from positions 2064 to 2074 in the nucleotide sequence of SEQ ID NO: 1;
(c) determining the molecular weight of the DNA amplified in step (b) by mass spectrometry; and
(d) comparing the molecular weight determined in step (c) with that of a control.

7. The method according to claim 1, comprising the steps of:
(a) preparing a DNA sample from a subject pig;
(b) amplifying a DNA that is a swine Mx1 gene exon and comprises the nucleotide sequence from positions 2064 to 2074 in the nucleotide sequence of SEQ ID NO: 1;
(c) preparing a substrate with an immobilized nucleotide probe;
(d) contacting the DNA prepared in step (b) with the substrate prepared in step (c);
(e) determining the intensity of hybridization between the DNA and the nucleotide probe immobilized on the substrate; and
(f) comparing the intensity determined in step (e) with that of a control.

8. The method according to claim 1, comprising the steps of:
(a) preparing a protein sample from a subject pig; and
(b) determining the amount of a mutant swine Mx1 protein in the protein sample, wherein said mutant swine Mx1 protein is encoded by a nucleotide sequence that is a swine Mx1 gene exon in which the 11-bp nucleotide sequence from positions 2064 to 2074 in SEQ ID NO: 1 has been deleted.

9. The method according to any one of claims 1 to 8, further comprising the step of determining that a subject pig is susceptible to an RNA virus when the 11-base deletion defined above is detected or the subject pig is resistant to the RNA virus when the deletion is not detectable.

10. The method according to any one of claims 1 to 9, wherein the RNA virus is an influenza virus or the causative virus of PRRS.

11. An oligonucleotide to be used as a PCR primer in the method according to any one of claims 1 to 10, wherein the oligonucleotide is used to amplify a DNA region that is a swine Mx1 gene exon and comprises the nucleotide sequence from positions 2064 to 2074 in the nucleotide sequence of SEQ ID NO: 1.

12. An oligonucleotide comprising at least 15 nucleotides, and hybridizing to a DNA region that is a swine Mx1 gene exon and comprises the nucleotide sequence from positions 2064 to 2074 in the nucleotide sequence of SEQ ID NO: 1, or a DNA region that is a swine Mx1 gene exon and comprises a nucleotide sequence in which the nucleotide sequence from positions 2064 to 2074 has been deleted.

13. An antibody recognizing a mutant swine Mx1 protein encoded by the nucleotide sequence of a swine Mx1 gene exon in which the nucleotide sequence from positions 2064 to 2074 in SEQ ID NO: 1 has been deleted.

14. A test reagent for determining a pig's resistance to an RNA virus, wherein the reagent comprises the oligonucleotide according to claim 11 or 12, or the antibody according to claim 13.

15. The test reagent according to claim 14, wherein the RNA virus is an influenza virus or the causative virus of PRRS.
